Europäisches Patentamt

European Patent Office

Office européen des brevets

(19)

(11) Veröffentlichungsnummer : **0 152 592**
**B1**

(12)

# EUROPÄISCHE PATENTSCHRIFT

(45) Veröffentlichungstag der Patentschrift :
19.08.87

(51) Int. Cl.⁴ : **A 61 B   6/04**, H 04 B   3/12

(21) Anmeldenummer : **84115377.8**

(22) Anmeldetag : **13.12.84**

(54) **Röntgengerät mit einer Patientenlagerungsplatte.**

(30) Priorität : **15.02.84 DE 3405425**

(43) Veröffentlichungstag der Anmeldung :
**28.08.85 Patentblatt 85/35**

(45) Bekanntmachung des Hinweises auf die Patenterteilung : **19.08.87 Patentblatt 87/34**

(84) Benannte Vertragsstaaten :
**DE FR GB IT**

(56) Entgegenhaltungen :
**EP-A- 0 023 554**
**BE-A-   688 786**
**DE-A- 1 964 292**
**US-A- 2 504 697**

(73) Patentinhaber : **Siemens Aktiengesellschaft Berlin und München**
**Wittelsbacherplatz 2**
**D-8000 München 2 (DE)**

(72) Erfinder : **Hahn, Alfred**
**Bunsenstrasse 64**
**D-8520 Erlangen (DE)**
Erfinder : **Giera, Manfred**
**Äussere Tennenloher Strasse 53**
**D-8520 Erlangen (DE)**
Erfinder : **Schäfer, Willi**
**Genglerstrasse 20**
**D-8520 Erlangen (DE)**

EP 0 152 592 B1

Jouve, 18, rue St-Denis, 75001 Paris, France

## Beschreibung

Die Erfindung bezieht sich auf ein Röntgengerät mit einer Patientenlagerungsplatte, die in Sandwichbauweise aufgebaut ist und unterhalb einer Deckplatte eine elektrische Heizvorrichtung aufweist.

Die bekannten Röntgengeräte fühlen sich für einen daraufgelegten Patienten inbesondere dann, wenn er für eine Röntgenuntersuchung mit der bloßen Haut mit der Patientenlagerungsplatte in Berührung kommt, häufig kalt an, was sehr störend ist.

Es ist demgemäß erwünscht, die Patientenlagerungsplatte zu beheizen. Hierzu sind durch die BE-PS 688 786 und die US-PS 2 504 697 Patientenlagerungsplatten der eingangs genannten Art bekannt, bei denen unterhalb der Deckplatte eine Heizschicht angeordnet ist. Die bekannten Patientenlagerungsplatten sind aus einer Vielzahl von Schichten aufgebaut und die Wärme der Heizvorrichtung wird sowohl nach oben als auch nach unten abgestrahlt.

Der Erfindung liegt die Aufgabe zugrunde, ein Röntgengerät der eingangs genannten Art so auszubilden, daß die von der Heizvorrichtung erzeugte Wärme im wesentlichen nur nach oben abgestrahlt wlrd, daß also der unterhalb der Heizvorrichtung liegende Teil der Patientenlagerungsplatte nur wenig erwärmt wird.

Diese Aufgabe ist erfindungsgemäß dadurch gelöst, daß die elektrische Heizvorrichtung zwischen der Deckplatte und einer zur thermischen Isolation dienenden Hartschaumschicht angeordnet ist. Durch die Heizvorrichtung kann die Temperatur der Patientenlagerungsplatte auf einen Wert angehoben werden, der für die Patienten angenehm ist. Untersuchungen haben gezeigt, daß ein praktisch sinnvoller Wert bei etwa 30° C liegt. Die Hartschaumschicht bewirkt dabei, daß die Wärme der Heizvorrichtung praktisch nur nach oben abgestrahlt wird, so daß sich die unter ihr liegenden Teile kaum erwärmen.

Die Erfindung ist nachfolgend anhand eines in der Zeichnung dargestellten Ausführungsbeispieles näher erläutert. Es zeigen :

Figur 1   ein Röntgendiagnostikgerät nach der Erfindung,

Figur 2   den Querschnitt und Aufbau der Patientenlagerungsplatte des Röntgendiagnostikgerätes gemäß Figur 1  in vergrößerter Darstellung, und

Figur 3   eine Draufsicht auf die geöffnete Patientenlagerungsplatte nach Figur 1.

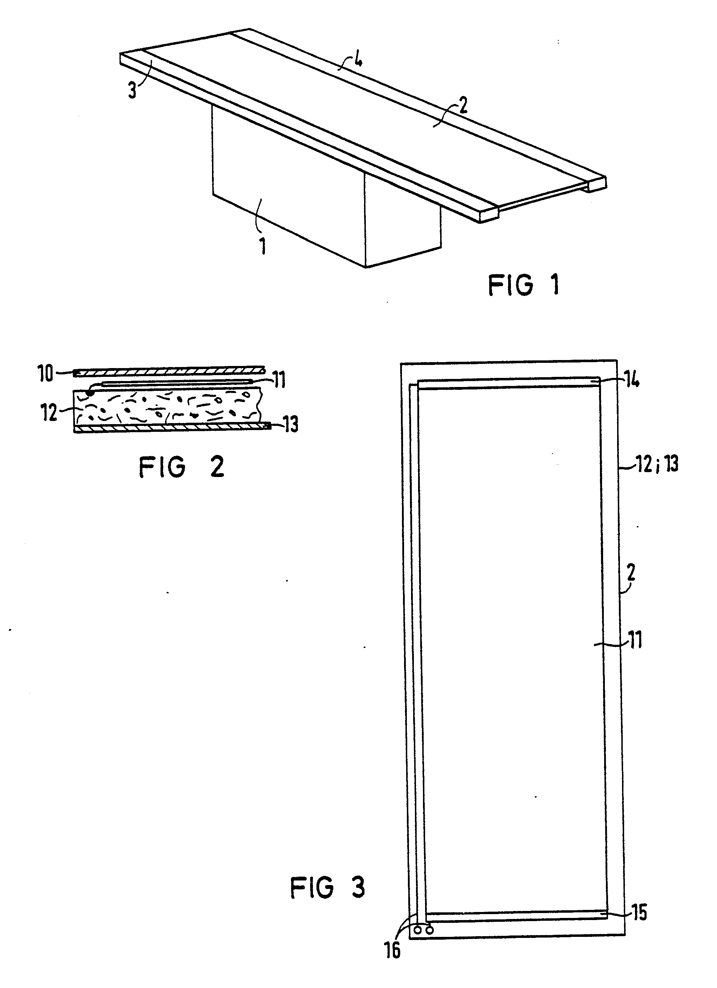

In der Figur 1  ist ein Röntgendiagnostikgerät mit einem Sockel 1 dargestellt, auf dem eine Patientenlagerungsplatte 2 längs- und querverschiebbar gelagert ist. Die Patientenlagerungsplatte 2 Ist zwischen zwei metallischen Längsholmen 3, 4 gehaltert, die mit Hilfe von Rollen auf Schienen verschiebbar geführt sind.

Die Figur 2  zeigt, daß die Patientenlagerungsplatte 2 in Sandwichbauweise aus einer Deckplatte 10, einer Heizfläche 11 und einem Hartschaum 12 besteht, der an seiner Unterseite von einer Grundplatte 13 abgedeckt ist. Die Heizfläche 11, die z. B. aus metallisiertem Gewebe bestehen kann, liegt unmittelbar unter der Deckplatte 10. Wird sie beispielsweise am oberen und am unteren Ende der Patientenlagerungsplatte 2 (Figur 1) kontaktiert und ein entsprechend hoher Strom durch die Heizfläche 11 geschickt, so wird die Patientenlagerungsplatte 2 beheizt. Die für eine angenehme Temperatur erforderliche Heizleistung für die Patientenlagerungsplatte 2 liegt dabei in der Größenordnung von etwa 200 W. Durch die gute thermische Isolationswirkung des Hartschaumes 12 wird praktisch die ganze Heizenergie zum Patienten hin abgeführt. Anstelle eines Heizgewebes kann auch eine Schicht aus elektrisch leitfähigem Lack auf der Rückseite der Deckplatte 10. , d. h. auf der dem Hartschaum 12 zugewandten Seite vorgesehen sein. Ferner kann auf dieser Seite eine dünne Metallschicht, z. B. aus Nickel, aufgedampft oder elektrolytisch aufgebracht sein, ebenso ist eine elektrisch leitende Kunststoffolie geeignet.

Wesentlich ist, daß die elektrische Heizfläche 11 homogen über die gesamte Patientenlagerungsplatte 2 verteilt ist und die Röntgenstrahlung nur unbedeutend schwächt, so daß keine störenden Strukturen in den Röntgenaufnahmen entstehen. Die von einer Widerstandsschicht gebildete Heizfläche 11 darf also nicht abgebildet werden.

Die Patientenlagerungsplatte 2 wird an den Anschlußstellen für die Energiezufuhr so ausgebildet, daß je nach Anwendungsfall Kabel oder Schleifkontakte eingesetzt werden können.

Die Figur 2  zeigt ein Ausführungsbeispiel, bei dem die Heizfläche 11 am oberen und unteren Ende über Kontaktstreifen 14, 15 kontaktiert ist. In der Patientenlagerungsplatte 2 verlaufen dabei die Zuleitungen 16 für die Stromzufuhr. Die Kontaktstreifen 14, 15 können von Metallstreifen, z. B. Kupferstreifen, mit U-förmigem Querschnitt gebildet sein, welche die Heizfläche 11 umfassen und gegebenenfalls mit dieser zur Reduzierung des Übergangswiderstandes auch verlötet sein können.

In ähnlicher Weise können allgemein Auflageflächen für Körperteile, z. B. für Mammographie und in der Chirurgie, mit Flächenheizelementen ausgestattet werden.

## Patentansprüche

1. Röntgengerät mit einer Patientenlagerungsplatte (2), die in Sandwichbauweise aufgebaut ist und unterhalb einer Deckplatte (10) eine elektrische Heizvorrichtung (11) aufweist, dadurch gekennzeichnet, daß die elektrische Heizvorrichtung (11) zwischen der Deckplatte (10) und einer zur thermischen Isolation dienenden Hartschaumschicht (12) angeordnet ist.

2. Röntgendiagnostikgerät nach Anspruch 1, dadurch gekennzeichnet, daß die Heizvorrichtung (11) von einem metallisierten Gewebe gebildet ist.

3. Röntgendiagnostikgerät nach Anspruch 1, dadurch gekennzeichnet, daß die Heizvorrichtung (11) von einer Schicht aus elektrisch leitfähigem Lack gebildet ist, der auf der Rückseite der Deckplatte (12) der Patientenlagerungsplatte aufgebracht ist.

4. Röntgendiagnostikgerät nach Anspruch 1, dadurch gekennzeichnet, daß die Heizvorrichtung (11) von einer dünnen Metallschicht gebildet ist, die auf der Rückseite der Deckplatte (12) der Patientenlagerungsplatte (2) aufgebracht ist.

## Claims

1. An X-ray apparatus comprising a support (2) for a patient, which is of sandwich construction and which comprises an electrical heating device (11) beneath a covering panel (10), characterised in that the electrical heating device (11) is arranged between the covering panel (10) and a thermally insulating hard foam layer (12).

2. An X-ray diagnostic apparatus as claimed in claim 1, characterised in that the heating device (11) is formed by a metallised fabric.

3. An X-ray diagnostic apparatus as claimed in claim 1, characterised in that the heating device (11) is formed by a layer of electrically conductive lacquer which is applied to the rear of the covering panel (10) of the support for the patient.

4. An X-ray diagnostic apparatus as claimed in claim 1, characterised in that the heating device (11) is formed by a thin metal layer which is applied to the rear of the covering panel (10) of the support for the patient.

## Revendications

1. Appareil radiologique comportant un plateau (2) formant couchette pour patient, qui possède une constitution en sandwich et comporte, au-dessous d'une plaque de revêtement (10), un dispositif de chauffage électrique (11), caractérisé par le fait que le dispositif de chauffage électrique (11) est disposé entre la plaque de revêtement (10) et une couche de mousse dure (12) servant à réaliser l'isolation thermique.

2. Appareil de radiodiagnostic suivant la revendication 1, caractérisé par le fait que le dispositif de chauffage (11) est formé par un tissu métallisé.

3. Appareil de radiodiagnostic suivant la revendication 1, caractérisé par le fait que le dispositif de chauffage (11) est formé par une couche de laque électriquement conductrice, qui est déposée sur la face arrière de la plaque de revêtement (12) du plateau formant couchette pour patient.

4. Appareil de radiodiagnostic suivant la revendication 1, caractérisé par le fait que le dispositif de chauffage (11) est formé par une couche métallique mince, qui est disposée sur la face arrière de la plaque de revêtement (12) du plateau (2) formant couchette pour patient.

FIG 1

FIG 2

FIG 3